# EUROPEAN PATENT APPLICATION

(11) **EP 2 246 044 A1**
(43) Date of publication of application: **03.11.2010**
(21) Application number: 09290298.0
(22) Date of filing: 21.04.2009
(51) Int. Cl.: A61K 9/00, A61K 31/13

(54) **Paediatric solutions comprising a beta-blocker**

(71) Applicant: Pierre Fabre Dermo-Cosmétique, 92100 Boulogne-Billancourt (FR)
(72) Inventor: Chaumont, Christine, 31400 Toulouse (FR); Cordoliani, Jean Francois, 31570 Sainte Foy D'Aigrefeuille (FR); Leverd, Elie, 81100 Castres (FR); Muguet, Valerie, 31400 Toulouse (FR)
(74) Representative: Hirsch & Associés

(57) **Abstract**

The present invention relates to aqueous non alcoholic solutions comprising a beta-blocker, a non sugar type sweetener and being substantially free of an aromatic preservative agent. The solutions of the present invention are particularly adapted for a paediatric use.

## Description

### FIELD OF THE INVENTION

The present invention relates to pharmaceutical solutions and more particularly to paediatric solutions comprising a beta-blocker such as propranolol or a pharmaceutically acceptable salt thereof.

### BACKGROUND OF THE INVENTION

Infantile capillary hemangiomas (IH) in children are commonly recognized in the skin and liver, and represent most common soft tissue tumours in 4 to 10% of children aged under 1 year. Despite their begnin self-limited course, common IH are rarely responsible during their proliferative phase for local complications such as ulceration or haemorrhage but may however impair in some cases vital or sensorial functions especially when present respectively on upper airways and orbital areas.

While the understanding of growth and involution of IH is still an issue, it has however been found an alternative treatment to the oral corticosteroids, vincristine or interferon alfa-1a and 2b, thereby avoiding constraining side effects in young patients. Because patients are infants or small children, patient tolerance to treatments is of the utmost importance.

As it was reported by C. Léauté-Labrèze et al. in Propranolol for severe hemangiomas of infancy, N. Engl. J. Chem. (2008) 358; 24: 2649-51, it has been observed and demonstrated that beta-blockers, e.g. propranolol, could efficiently be used to control and even to treat the growth of IH.

Propranolol, (1-(isopropylamino)-3-(naphthalen-1-yloxy)propan-2-ol), is a well tolerated non-selective beta blocker commonly used in young children for cardiologic indications as disclosed in several publication e.g by Villain et al. in Low incidence of cardiac events with beta-blocking therapy in children with long QT syndrome, Eur Heart J. 2004, 25:1405-11; by Fritz et al in Effect of beta-blockade on symptomatic dexamethasone-induced hypertrophic obstructive cardiomyopathy in premature infants: three case reports and literature review. J. Perinatol 1998; 18:38-44 and by Kilian K. in Hypertension in neonates causes and treatments, J Perinat Neonatal Nurs, 2003; 17:65-74.

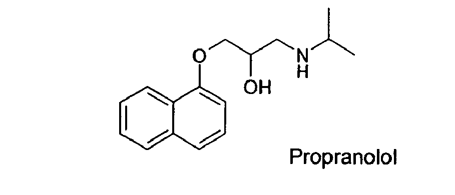

Propranolol is particularly available on the market under different formulations in the Syprol® product, the Avlocardyl® product or the drinkable solution of Roxane Laboratories. However, the existing solutions are not appropriate for a paediatric use. For example, the solution of Roxanne Laboratories contains alcohol (0.6%) which is particularly toxic in young patients.

Several aqueous solutions comprising propranolol have already been disclosed. In FR 2 688 405 for example, sustained release formulations for the oral delivery of beta blockers such as propranolol are prepared with polysaccharides and further comprise a preservative agent such as parabens. US 4,600,708 discloses liquid therapeutic dosage formulations of propranolol HCl containing lecithin and all of the disclosed examples contain methyl- and propylparaben. EP 732 064 relates to a bitterness-relieving agent which comprises an ester of a mono or diglyceride with a polycarboxylic acid salt. It is used to mask propranolol HCl in a liquid solution but however also encompasses a butyl-parabens excipient. Such preservative agents are not appropriate for a paediatric use.

Extemporaneous solutions of propranolol are also known. These were prepared by dissolving crushed tablets of propranolol in aqueous solutions together with preservative agents such as parabens or aromatic type preservatives. Indeed, their use is usually recommended as reported by D. Woods in "Extemporaneous formulations of Oral Liquids, a guide*".* Other known extemporaneous solutions for the oral administration of propranolol are syrup types which are thus based on a sucrose vehicle, which cannot be adapted for paediatric patients. Such syrups are provided for example in *"*Formulation in Pharmacy Practice" 2nd ed (www.pharminfotech.co.nz), or by V. Das Gupta in "Stability of Propranolol Hydrochloride Suspension and solution compounded from injection or tablets", Am. J. of Hospital Pharmacy, (1987) 44:360-361.

However, while numerous formulations of beta-blockers already exist in the art, none of them are suitable for paediatric patients since they contain alcoholic excipients or aromatic preservative agents such as benzyl alcohol that need to be carefully evaluated in young patients up to three years old. Furthermore, it has also been established that benzoic acid, sodium benzoate and potassium benzoate may increase the risk of jaundice in neonates. Similarly, ethanol is to be excluded. Thus, because aromatic preservatives and alcoholic excipients must not be given to neonates and young infants due to their immature metabolism, there has always been a need to provide safe and suitable compositions or formulations for the paediatric administration of beta-blockers.

### SUMMARY OF THE INVENTION

According to one aspect, the present invention is directed to an aqueous non alcoholic solution comprising a beta-blocker, a non sugar type sweetener and being substantially free of an aromatic preservative agent.

According to one embodiment, the at least one non-sugar type sweetener is selected from saccharin, saccharin salts, sodium saccharin, calcium saccharin, sucralose, potassium acetosulfam, stevioside, steviol, mannitol, erythritol, lactitol, alitame, miraculin, monellin, thaumatin, and mixture thereof, and for example selected from saccharin sodium.

According to one embodiment, the at least one beta-blocker is selected from non-selective betablocker, for example from alprenolol, bucindolol, carteolol, carvedilol, labetalol, levobunolol, medroxalol, mepindolol, metipranolol, nadolol, oxprenolol, penbutolol, pindolol, propafenone, propranolol, sotalol, timolol, pharmaceutically acceptable salts thereof, and e.g. propranolol or pharmaceutically acceptable salt thereof

According to one embodiment, the aqueous solution further comprises at least one non-aromatic preservative agent, at least one flavouring agent, a buffer and/or at least one viscosity increasing agent.

According to one embodiment, the at least one non-aromatic preservative agent is selected from propionic acid or pharmaceutically acceptable salts thereof, sorbic acid or pharmaceutically acceptable salts thereof, and mixtures thereof, for example in an amount of from 0 to 1% w/V, and e.g. of from 0 to 0.5% w/V.

According to one embodiment, the at least one flavouring agent is selected from any of the cherry, lemon, lime, mandarin, orange, tangerine, mint, strawberry, banana, caramel, liquorice, passion-fruit, peach, raspberry, tutti-frutti, grapefruit, vanilla, cream, chocolate, grape flavour or mixture thereof, for example selected from the vanilla and strawberry flavour, and for example in an amount of from 0 to 5% w/V, e.g. of from 0.05 to 1% w/V and e.g. of from 0.05 to 0.5 % w/V.

According to one embodiment, the at least one viscosity increasing agent is selected from cellulose derivatives, hydroxyethylcellulose, hydroxypropylcellulose, hydroxypropylmethylcellulose, or methylcellulose, poloxamers, from gums, guar gum, tragacanthe gum, acacia gum, xanthane gum, alginic derivatives, alginic acid, sodium alginate, polyvinylpyrrolidone, from silicates, bentonite, laponite, veegum, for example hydroxyethylcellulose, and is for example in an amount of from 0 to 15% w/V, e.g. of from 0.1 to 10% w/V, e.g. of from 0.1 to 5 % w/V and e.g. of from 0.1 to 0.5%w/V.

According to another aspect, the present invention is directed to an aqueous non alcoholic solution consisting of essentially a beta-blocker, non-sugar type sweetener(s), flavouring agent(s), up to 1% w/V of non-aromatic preservative(s) and optionally viscosity increasing agent(s).

According to one embodiment, the beta-blocker is in an amount of from 0.01 to 20% w/V, for example of from 0.05 to 10% w/V, e.g. of from 0.1 to 5 % w/V and e.g. of from 0.1 to 1% w/V*,* the at least non-aromatic preservative is in an amount of from 0 to 1% w/V, for example of from 0 to 0.5% w/V, the at least one non-sugar type sweetener is in an amount of from 0.01 to 5% w/V, for example of from 0.05 to 1% w/V, e.g. of from 0.05 to 0.5 % w/V and e.g. of from 0.05 to 0.2%w/V, the at least one flavouring agent is in an amount of from 0 to 5% w/V, for example of from 0.05 to 1% w/V and e.g. of from 0.05 to 0.5 % w/V, the at least one viscosity increasing agent is in an amount of from 0 to 15% w/V, for example of from 0.1 to 10% w/V, e.g. of from 0.1 to 5 % w/V and e.g. of from 0.1 to 0.5%w/V.

According to one embodiment, the beta-blocker is propranolol hydrochloride in an amount of 0.57 % w/V, the at least one sweetener is saccharin sodium in an amount of 0.15% w/V, the at least one flavouring agent is a mixture of vanilla and strawberry flavour in an amount of 0.32% w/V the at least one viscosity increasing agent is hydroxyethylcellulose in an amount of 035 % w/V, and optionally comprising sodium propionate in an amount of 0.10% w/V. According to one embodiment of the present invention, the pH is comprised between 2 and 5.5, for example between 2.5 and 4.

According to one embodiment, the solution is a multi-use solution.

According to one embodiment, the solution is suitable for oral administration.

According to another aspect, the present invention is directed to a process for the preparation of the aqueous solutions of the invention, comprising the step of dissolving the beta-blocker into a solvent, for example water.

According to another aspect, the present invention is directed to a solution of the invention for use as a medicament in the treatment of hemangiomas, for example capillary hemangiomas and e.g. capillary infantile hemangiomas.

According to another aspect, the present invention is directed to the use of a beta-blocker, for example propranolol as a preservative agent.

According to another aspect, the present invention is directed to a device comprising a container and a scale marked pipette or oral syringe indicating doses based on the body weight of the patient, containing the solution of the invention.

According to one embodiment, the scale marks each 0.1 ml doses.

### DETAILLED DESCRIPTION OF THE INVENTION

It has been found in a surprising manner that beta-blockers such as propranolol provide a preservative effect that could be used in formulations intended for paediatric patients. While conventional aromatic preservative agents should be excluded from paediatric products due to their toxic effects on immature metabolisms, these products should also fulfil the preservation requirements against micro-organism proliferation. This has been achieved in the present invention, based on the surprising discovery that beta-blockers such as propranolol provide suitable preservative and stability properties to aqueous solutions that are suitable for oral administration to young patients.

The solutions of the present invention are pharmaceutically acceptable and will be particularly adapted for multiple uses, having appropriate storage capabilities.

A most useful application is found in the treatment of infantile hemangiomas.

By 'pharmaceutically acceptable' is meant a material that is not biologically or otherwise undesirable, e.g. the material may be incorporated into a dosage form of the invention without causing any undesirable biological effects or interacting in a deleterious manner with any of the other components of the dosage form formulation.

According to one embodiment, the present invention is directed to an aqueous solution of a beta-blocker such as propranolol, being substantially free of alcohol and aromatic preservative agents.

As given in the recommendations of the reflection paper of the Committee for medicinal products for human use at the EMEA (EMEA/CHMP/PEG/194810/2005), oral administration is commonly used for paediatric patients, and liquid formulations are best suited for administration to young infants who are unable to swallow capsules or tablets.

Such aqueous solutions also include suspensions or emulsions that would fall within the scope of the present invention. They may be applied by instillation, spraying, squirting into the oral cavity of the patient, or mixed with milk or fruit juice before administration.

The term 'beta-blockers', refers to beta-receptor blocking agent, beta adrenergic receptor blocking agent, beta blocking agent, beta-blocking agent, beta-blocking agent or beta-adrenergic receptor blocking agent or any other denomination indicating a chemical that inhibits the binding of agonists, natural or artificial, to beta-adrenergic receptors of any type (bet-1, beta-2, beta-3 or others).

According to the present invention the beta-blocker may be a non-selective beta-blocker, a beta-1-selective beta-blocker, a mixture of alpha-1/beta-adrenergic antagonists, a beta-2selective beta-blocker. It may also be a mixture of to or more beta-blockers.

For example, formulations within the scope of the invention are best suited for non-selective beta-blocker, such as alprenolol, bucindolol, carteolol, carvedilol, labetalol, levobunolol, medroxalol, mepindolol, metipranolol, nadolol, oxprenolol, penbutolol, pindolol, propafenone, propranolol, sotalol, Timolol.

For example the beta-blocker is propranolol which may be present in the L or D-propranolol form, or racemate, or in one of its pharmaceutically acceptable salts such as the hydrochloride salt.

The beta-blocker that is used will be present in the solution in an amount of from 0.01 to 20% w/V, e.g. from 0.05 to 10% w/V and e.g. from 0.1 to 5 % w/V and for example from 0.1 to 1% w/V.

The aqueous solutions of the invention may further contain a buffer in order to provide the appropriate pH to the solution for the beta-blocker. For example, an appropriate pH for propranolol would be of about 3 to 6, provided by the use of a suitable buffering agent such as mineral acids such as hydrochloric acid or organic acids such as citric acid, or buffer mixtures such as citric acid/sodium citrate, acetic acid/sodium acetate.

The present invention is based on the surprising and unexpected discovery that aqueous solutions comprising a beta-blocker such as propranolol could be free of alcohol and aromatic preservative agents although such compositions are intended for a pharmaceutical use. Particularly, the present invention provides aqueous solutions of beta-blockers such as propranolol that are substantially free of any preservative agents and alcohol. These solutions are suitable for multiple use, i.e. the solutions can be stored between different administrations steps where administration must be repeated, e.g. once or twice daily.

Because paediatric products are intended to patients who have limited immune defences, the tendency is to take all the precautionary measures in order to avoid any bacterial infection. It is thus particularly surprising that aqueous solutions being substantially free of aromatic preservatives (and in some cases substantially free of preservatives at all) and alcohol could be safely preserved and suitably administered to infants.

The term "aromatic preservative agent" encompasses (besides the beta-blocker itself), any agent that is usually added to avoid the growth of microorganisms or avoid undesirable side reactions. Examples of preservative agents that are conventionally met, are benzalkonium chloride, benzethonium chloride, benzoic acid, benzyl alcohol, cetylpyridinium chloride, phenoxyethanol, cresol, phenol, phenylethyl alcohol, phenylmercuric acetate or nitrate, sodium benzoate, thimerosal, thymol compounds or compounds of the parabens family such as methylparaben, ethylparaben, propylparaben, butylparaben, and isobutylparaben.

The term "alcoholic" refers to alcohols for example ethanol.

By avoiding aromatic preservatives, the aqueous solutions according to the present invention may comprise preservative agents that are safe for paediatric patients. This includes for example the use of chlorobutanol, dehydroacetic acid, sodium dehydroacetate, propionic acid, propionate salt, such as sodium propionate or potassium propionate, sorbic acid, sorbate salt such as potassium sorbate or sodium sorbate, or mixtures thereof. Typically the appropriate amount of non-aromatic preservative present in the aqueous solution may be in an amount of from 0 to 1% w/V, e.g. of from 0 to 0.5% w/V.

The term "substantially free" implies that the solutions within the scope of the invention should be as free of aromatic preservative agent as it is practically and realistically feasible. This covers notably compositions comprising less than 0,01w/V% of the compound of interest.

Compositions according to the invention appeared to remain stable, preserved from contamination and thus suitable for a paediatric use.

The aqueous solutions of the invention also include one or more sweetening agents that are specifically of a non-sugar type. These are incorporated into the formulation in order to enhance the taste of the dosage forms and provide compositions that will be edible by the young patients. Sweeteners notably mask the bitterness and anaesthesic effect of propranolol. Indeed, paediatric patients are able to recognize sweetness from an early stage of life but also to recognize sweet taste in mixtures and estimate the strength or degree of sweetness.

Any artificial or organic non-sugar type sweetener may be used besides those that are unsuitable for paediatric products. The term "sugar type sweetener" encompasses monosaccharides or disaccharides such as e.g. sucrose, fructose, glucose or dextrose. Sucrose, which is the most commonly used sweetening agent is a disacharride that is hydrolysed in the intestine to the absorbable monosacharride fructose and glucose.

The term "non-sugar" encompasses artificial sweetening agents that reduce the caloric intake such as, for instance, any of saccharin, saccharin salts, e.g., sodium saccharin, calcium saccharin, sucralose, potassium acetosulfam, stevioside, steviol, mannitol, erythritol, lactitol, alitame, miraculin, monellin, and thaumatin.

Non sugar type sweetening agents are preferred in the instant invention since some patients may suffer hereditary fructose intolerance or diabetes, while it has been established that sucrose causes a decrease in dental plaque pH, the dissolution of tooth enamel, and promotes dental caries. It has also been observed that fructose causes laxative effects at high doses.

A most preferred non sugar sweetening agent is saccharine and for example saccharine sodium or calcium salt.

Aspartame, xylitol, maltitol and sorbitol are preferably excluded from the instant solutions. Both sorbitol and xylitol may cause osmotic diarrhea while sorbitol is degraded into fructose and thus represents potential risks to patients who have hereditary fructose intolerance and hypoglycaemia. Aspartame, may be harmful in patients with phenylketonuria and is usually contra-indicated in homozygous automosal recessive patients. Therefore, these components are preferably excluded from the present invention.

The sweetening agent should be present in an amount that is in accordance with safety recommendations and that provides a taste-masking efficacy but in a sufficiently low amount no to develop well-known bitter taste, as it is the case for saccharine.

Amounts of sweetening agents may be typically comprised in the range of from 0.01 to 5% w/V, for example 0.05 to 1% w/V and for example from 0.05 to 0.5 % w/V and for example from 0.05 to 0.2%w/V.

In certain embodiments, the present invention may further comprise flavouring agents which have the advantage of enhancing the potential acceptance of the composition by the child, but also have the advantage of providing a taste-masking effect for the other excipients that are found in the composition. The appropriate type and amount of flavour depends on social and cultural factors, such as food selection of adults which may have affected the flavour preferences of children.

Suitable flavours can be organic or artificial flavours it they are food grade certified by the manufacturer and have approved by the competent regulatory authorities in order to be adapted for the instant embodiment.

Examples of appropriate flavours would then be any that tastes cherry, lemon, lime, mandarin, orange, tangerine, mint, strawberry, banana, caramel, liquorice, passion-fruit, peach, raspberry, tutti-frutti, grapefruit, vanilla, cream, chocolate, grape or mixture thereof. Considering the bitter taste of beta-blockers, and more particularly of propranolol, the red or yellow fruit flavours as well as the chocolate, vanilla, caramel are preferred.

For example the strawberry and vanilla flavours appeared to be particularly accepted by the patients. Furthermore, the vanilla flavour has also recognized preservative effects and enhances the sweetness of the formulation, while the strawberry is particularly suitable to mask the bitterness of the beta-blocker such as propranolol.

Amounts of flavouring agents may typically be comprised in the range of from 0 to 5% w/V, for example from 0.05 to 1% w/V and e.g. from 0.05 to 0.5 % w/V %.

It should be noted that some flavouring agents may be dissolved in propylene glycol. Products containing high levels of propylene glycol should not be administered to paediatric patients below the age of 4 years (EMEA/CHMP/PEG/194810/2005). According to the present invention, flavouring agents provide an amount of propylene glycol which is below this level. For example, an amount of propylene glycol as low as of 0.23% w/V is acceptable for paediatric patients and is obtained by the use of strawberry and vanilla flavours in a respective amount of 0.11 % w/V and 0.21 %wN as disclosed in the formulations of example 1.

According to another embodiment, compositions of the present invention may further comprise a viscosity increasing agent in order to enhance the palatability of the solution, emulsion or suspension. A thickened liquid may also be particularly convenient and adapted to be administered to infants to avoid spillage of solutions. Furthermore, it also has the advantage to facilitate the manipulation of the solution out of the container when using a pipette or an oral syringe

Examples of thickener agents that can be used in the present invention are the celluloses derivatives such as hydroxyethylcellulose, hydroxypropylcellulose, hydroxypropylmethylcellulose, methylcellulose, gums, guar gum, tragacanthe gum, acacia gum, xanthan gum, alginic derivatives, alginic acid, sodium alginate, polyvinylpyrrolidone, silicates, bentonite, laponite, veegum. Poloxamers can also show thickening properties. Preferred viscosity increasing agents is hydroxyethylcellulose since it is particularly suitable for aqueous solutions.

Typically, the amount of viscosity increasing agent may be comprised from 0 to 15% w/V, for example from 0.1 to 10% w/V, e.g. from 0.1 to 5 % w/V % and e.g. from 0.1 to 0.5%w/V.

The compositions of the instant invention can further comprise any other excipient that would be suitable for a pharmaceutical use. This includes for example, solubility enhancing agents, buffers, colouring agents emulsifiers and solvents. Further examples of conventional buffers and excipients that may be considered by a person skilled in the art can be found in 'Handbook of Pharmaceutical Excipients'; Ed. A.H. Kibbe, 3rd Ed., American Pharmaceutical Association, USA and Pharmaceutical Press UK, 2000, the teaching of which are incorporated herein by reference.

The compositions and formulations within the scope of the invention may conveniently be prepared from conventional processes, involving the dissolution of at least one beta-blocker such as propranolol into a suitable solvent e.g. purified water.

Suitable processes may more conveniently be conducted according to the quality standards that are defined for example in the Note for Guidance on process for validation (EMEA/CVMP/598/99) and annex II to the Note for Guidance on process validation (CHMP/QWP/848/99) in order to obtain validation of the manufacturing process in a marketing authorisation.

According to another aspect, the invention is also directed to a device comprising a container with the aqueous solutions according to the invention and a graduated or scaled marked pipette or oral syringe indicating doses in relation to the body weight of the patient. This pipette will for example indicate doses in relation to the body weight of the patient. This device will be used for the storage and convenient delivery of the liquid into the buccal cavity of the child.

A plastic material is particularly suited for the pipette. Because it is usually difficult to evaluate the appropriate dose that should be administered without risks of overdoses (especially in the case where the active ingredient is a beta-blocker), this container and graduated pipette provides visual indications that allows an exact dosage directly based on the weight of the patient.

The container can be made out of a plastic or glass bottle, glass, for example amber glass in order to avoid unnecessary degradation from light of the beta-blocker during storage.

Since the product is intended for paediatric patients, the container should ideally be closed with a child resistant closure in order to render the content of the container inaccessible to infants.

### Example 1: Aqueous solutions within the scope of the invention.

| | 1 | 2 | 3 | 4 |
|---|---|---|---|---|
| propranolol HCl | 0.143 g | 0.285 g | 0.428 | 0.57 g |
| *corresponding to propranolol* | *0.125 g* | *0.250 g* | *0.375 g* | *0.50* g |
| hydroxyethyl cellulose | 0.35 g | | | 0.35 g |
| sodium Saccharin | 0.15 g | | | 0.15 g |
| sodium propionate | 0.10 g | | | - |
| strawberry flavour | 0.11 g | | | 0.11 g |
| vanilla flavour | 0.21 g | | | 0.21 g |
| (propylene glycol from flavours) | 0.23 g | | | 0.23 g |
| monohydrated citric acid | pH=3 | | | pH=3 |
| Purified water | QSP 100 ml | | | QSP 100 ml |

The solutions were prepared by placing most of the purified water which is necessary into an appropriate stainless steel tank. Under stirring, the following components were added and dissolved: a. hydroxyethylcellulose, b. saccharin sodium, c. propranolol hydrochloride, d. strawberry flavour, e. vanilla flavour. The pH of the solution was then adjusted to the pH value of 3.0 with an aqueous solution of monohydrated citric acid. Finally, the bulk was brought to the final volume with the remaining amount of water and well mixed.

Once filtered, the prepared solutions can be filled into the amber glass bottles and closed with a child resistant cap.

### Example 2: Microbiological data

The following formulations were tested to different strains in order to determine their microbiological resistance.

| Components | For 100 ml | |
|---|---|---|
| | Solution A | Solution B |
| Propranolol hydrochloride | 0.50 g | 0.50 g |
| Natrosol HHX250 | 0.35g | 0.35g |
| (hydroxyethylcellulose) | | |
| Sodium Saccharine | 0.15g | 0.15g |
| Hydrophilic flavour | 0.5g (vanilla) | 0.5g (caramel/vanilla) |
| Citric acid monohydrated | Qs pH =5.00 | Qs pH =3.00 |
| Preservative | No | Methylparaben 0.1 g |
| Purified water | Qs 100 ml | Qs 100 ml |

In the following tests, each test products were inoculated with 200 µl of each test strain diluted in tryptone salt. Microbiological countings were performed at day 0, 14 and 28, after that the samples were neutralized in 9 ml of a neutralizing solution (10 minutes) comprising tween 80 (10%), lecithin (2%) Saponin thiosulfate (0.5%) and sterile distilled water, and that 1 ml of that neutralized mixture was transferred in Petri dishes and covered with 15 ml of agar medium (Medium based on trypcase soy agar in case for aerobic bacteria and medium based on sabouraud dextrosed agar for yeasts and moulds). Countings were achieved after 24-48 hours incubation at 32.5°C ± 2.5°C for aerobic bacteria, and 48-72 hours at 22.5°C ± 2.5°C for yeasts and moulds. For each tested solution, the following results indicate the number of colony forming unit per ml, i.e. CFU/ml of test product and the ratio of reduction (in log) in comparison to the number of micro-organisms introduced in the inoculum.The results are expressed in the below table in CFU/ml of test product and in logarithmic reduction

The tested samples were all tested to the following micro-organisms: *Staphylococcus aureus, Pseudomonas aeruginosa, Escherichia coli, Candida albicans, and Aspergillus Niger.*

For each tested solution, the following results indicate the number of recovered micro-organisms towards the different strains, as well as the ration of reduction (in log) in comparison to the number of micro-organisms introduced in the inoculum. Typically, a reduction of from > 4.7 log or more indicates the complete disappearance of micro-organisms, due to the sensibility of the apparels.

### Solution A.

| strain | Inoculum | D0 | 14 days | | 28 days | | Accordance with Eur Ph. criteria |
|---|---|---|---|---|---|---|---|
| S. aureus | 9.8.10⁵ | 1.10⁶ | < 10 | > 5 log | < 10 | > 5 log | + |
| P. aeruginosa | 5.10⁵ | < 10 | < 10 | > 4.7 log | < 10 | > 4.7 log | + |
| E. coli | 1.3.10⁶ | 2.8.10⁵ | < 10 | > 5 log | < 10 | > 5 log | + |
| C. albicans | 0.9.10⁵ | 1.7.10⁵ | < 10 | > 4 log | < 10 | > 4 log | + |
| A. Niger | 1.3.10⁵ | 2.9.10⁵ | 6.8.10³ | 1.3 log | 5.3.10³ | 1.4 log | + |

### Solution B.

| strain | Inoculum | D0 | 14 days | | 28 days | | Accordance with Eur Ph. criteria |
|---|---|---|---|---|---|---|---|
| S. aureus | 9.8.10⁵ | 8.6.10⁶ | < 10 | > 5 log | < 10 | > 5 log | + |
| P. aeruginosa | 5.10⁵ | < 10 | < 10 | > 4.7 log | < 10 | > 4.7 log | + |
| E. coli | 1.3.10⁶ | 8.5.10⁵ | < 10 | > 5 log | < 10 | > 5 log | + |
| C. albicans | 0.9.10⁵ | 1.3.10⁵ | < 10 | > 4 log | < 10 | > 4 log | + |
| A. Niger | 1.3.10⁵ | 3.4.10⁵ | 4.1.10³ | 1.5 log | 1.8.10³ | 1.9 log | + |

Recommended criteria (European Pharmacopoeia 6^{th} edition (2008)) (Chap.5.1.3) for oral formulations.

| T0 + 14 days | T0 + 28 days | |
|---|---|---|
| Bacteria | 3 log | No increase |
| Moulds and yeasts | 1 log | |

Recommended criteria (US Pharmacopeia) for oral formulations

| T0 + 14 days | T0 + 28 days | |
|---|---|---|
| Bacteria | of 1 log | No increase |
| Moulds and yeasts | No increase | |

Thus, it clearly appears from the recommended criteria (European Pharmacopeia Chap 5.1.3) for oral formulations that the formulations of the invention remain adapted against microbiological development.

### Example 3:

Based on a similar assay as in example 2, the preservative effect of propranolol was demonstrated by testing the following compositions, Solution C and Solution D, to the previous strains.

| Components | For 100 ml | |
|---|---|---|
| | Solution C | Solution D |
| Propranolol hydrochloride | Placebo | 0.50 g |
| Natrosol HHX250 | - | - |
| (hydroxyethylcellulose) | | |
| Sodium Saccharine | - | - |
| Preservative | No | No |
| Citric acid monohydrated | Qs pH =2.84 | Qs pH =3.07 |
| Purified water | Qs 100 ml | Qs 100 ml |

| Solution C | T0 + 14 days | T0 + 28 days | Solution D | T0 + 14 days | T0 + 28 days |
|---|---|---|---|---|---|
| Aerobic bacteria | > 4.8 log | > 4.8 log | Aerobic bacteria | > 4.8 log | > 4.8 log |
| Fungic germs | Ca: 1.6 log | Ca: 1.6 log | Fungic germs | Ca : > 4.9 log | Ca : > 4.9 log |
| | An: 0.6 log | An : 1.5 log | | An : 3.2 log | An : 2.6 log |

Ca refers to the *Candida albicans* micro-organisms and An to the *Aspergillus Niger* micro-organisms.

The results indicate that the formulation with the placebo and without preservative agent was not acceptable under any of the US or the European Pharmacopeia recommendations after 14 days. The composition containing propranolol, e.g. 0.5g/100 ml without preservatives, however fulfilled the criteria even after 28 days.

For each tested solution, the following results indicate the ratio of reduction (in log) in comparison to the number of micro-organisms introduced in the inoculum.

### Example 4: Clinical Protocol

1) Diagnosis and criteria for inclusion :A subject is eligible if he/she meets all of the following criteria: 1) Written informed consents for study participation and the use of the subject's images are obtained from the subject's legal parental custodian(s) prior to performing any study procedures., 2) The subject is 35 to 150 days old, inclusive, at inclusion., 3) A large facial proliferating infantile hemangioma requiring systemic therapy is present, especially one with a prominent
2) Mode of administration: Administration of propranolol oral solution according to Example 1 above, formula 1, 2 and 3 of example 1, twice daily (morning and late afternoon) for 3 or 6 months
3) Titration procedure: D0 1 mg/kg/day, D7 increase to 2 mg/kg/day (for the 3 mg/kg/day arm), D14 increase to 3 mg/kg/day (for the 3 mg/kg/day arm).
4) Treatment duration: Propranolol is administered for 3 months or 6 months, depending on the assigned regimen.
5) Evaluation criteria:
   The primary efficacy criterion is the evolution of target IH from baseline to W24.

## Claims

1. An aqueous non alcoholic solution comprising a beta-blocker, a non sugar type sweetener and being substantially free of an aromatic preservative agent.

2. The aqueous solution according to claim 1, wherein the at least one non-sugar type sweetener is selected from saccharin, saccharin salts, sodium saccharin, calcium saccharin, sucralose, potassium acetosulfam, stevioside, steviol, mannitol, erythritol, lactitol, alitame, miraculin, monellin, thaumatin, and mixture thereof, and for example selected from saccharin sodium.

3. The aqueous solution according to anyone of claims 1 or 2, wherein the at least one beta-blocker is selected from non-selective betablocker, for example from alprenolol, bucindolol, carteolol, carvedilol, labetalol, levobunolol, medroxalol, mepindolol, metipranolol, nadolol, oxprenolol, penbutolol, pindolol, propafenone, propranolol, sotalol, timolol, pharmaceutically acceptable salts thereof, and e.g. propranolol or pharmaceutically acceptable salt thereof

4. The aqueous solution according to anyone of claims 1 to 3, further comprising at least one non-aromatic preservative agent, at least one flavouring agent, a buffer and/or at least one viscosity increasing agent.

5. The aqueous solution according to claim 4, wherein the at least one non-aromatic preservative agent is selected from propionic acid or pharmaceutically acceptable salts thereof, sorbic acid or pharmaceutically acceptable salts thereof, and mixtures thereof, for example in an amount of from 0 to 1% w/V, and e.g. of from 0 to 0.5% w/V.

6. The aqueous solution according to claim 4 or 5, wherein the at least one flavouring agent is selected from any of the cherry, lemon, lime, mandarin, orange, tangerine, mint, strawberry, banana, caramel, liquorice, passion-fruit, peach, raspberry, tutti-frutti, grapefruit, vanilla, cream, chocolate, grape flavour or mixture thereof, for example selected from the vanilla and strawberry flavour, and for example in an amount of from 0 to 5% w/V, e.g. of from 0.05 to 1% w/V and e.g. of from 0.05 to 0.5 % w/V.

7. The composition according to any one of claims 4 to 6, wherein the at least one viscosity increasing agent is selected from cellulose derivatives, hydroxyethylcellulose, hydroxypropylcellulose, hydroxypropylmethylcellulose, or methylcellulose, poloxamers, from gums, guar gum, tragacanthe gum, acacia gum, xanthane gum, alginic derivatives, alginic acid, sodium alginate, polyvinylpyrrolidone, from silicates, bentonite, laponite, veegum, for example hydroxyethylcellulose, and is for example in an amount of from 0 to 15% w/V, e.g. of from 0.1 to 10% w/V, e.g. of from 0.1 to 5 % w/V and e.g. of from 0.1 to 0.5% w/V.

8. An aqueous non alcoholic solution consisting of essentially a beta-blocker, non-sugar type sweetener(s), flavouring agent(s), up to 1% w/V of non-aromatic preservative(s) and optionally viscosity increasing agent(s).

9. The composition according to anyone of claims 1 to 8, wherein the beta-blocker is in an amount of from 0.01 to 20% w/V, for example of from 0.05 to 10% w/V, e.g. of from 0.1 to 5 % w/V and e.g. of from 0.1 to 1% w/V, wherein the at least non-aromatic preservative is in an amount of from 0 to 1 % w/V, for example of from 0 to 0.5% w/V, wherein the at least one non-sugar type sweetener is in an amount of from 0.01 to 5% w/V, for example of from 0.05 to 1% w/V, e.g. of from 0.05 to 0.5 % w/V and e.g. of from 0.05 to 0.2%w/V, wherein the at least one flavouring agent is in an amount of from 0 to 5% w/V, for example of from 0.05 to 1% w/V and e.g. of from 0.05 to 0.5 % wN , the at least one viscosity increasing agent is in an amount of from 0 to 15% w/V, for example of from 0.1 to 10% w/V, e.g. of from 0.1 to 5 % w/V and e.g. of from 0.1 to 0.5%w/V.

10. The composition according to claim 9, wherein the beta-blocker is propranolol hydrochloride in an amount of 0.57 % w/V wherein the at least one sweetener is saccharin sodium in an amount of 0.15% w/V, wherein the at least one flavouring agent is a mixture of vanilla and strawberry flavour in an amount of 0.32% w/V the at least one viscosity increasing agent is hydroxyethylcellulose in an amount of 035 % w/V, and optionally comprising sodium propionate in an amount of 0.10% w/V.

11. The solution according to anyone of claims 1 to 10, wherein the pH is comprised between 2 and 5.5, for example between 2.5 and 4.

12. The solution according to anyone of claims 1 to 11, wherein the solution is a multi-use solution.

13. The solution according to anyone of claims 1 to 12, which is suitable for oral administration.

14. A process for the preparation of the aqueous solutions of according to anyone of claims 1 to 13, comprising the step of dissolving the beta-blocker into a solvent, for example water.

15. The solution according to anyone of claims 1 to 13, for use as a medicament in the treatment of hemangiomas, for example capillary hemangiomas and e.g. capillary infantile hemangiomas.

16. The use of a beta-blocker, for example propranolol as a preservative agent.

17. A device comprising a container and a scale marked pipette or oral syringe indicating doses based on the body weight of the patient, containing the solution according to anyone of claims 1 to 13.

18. A device according to claim 17, where the scale marks each 0.1 ml doses.
